# EUROPEAN PATENT APPLICATION

(11) **EP 4 850 726 A2**
(43) Date of publication of application: **09.12.2026**
(21) Application number: 26214532.9
(22) Date of filing: 06.12.2024
(51) Int. Cl.: A61M 15/00

(54) **DRIVER FOR MIST GENERATOR**

(30) Priority: 06.12.2023 US 202363606859 P
(62) Document number(s) of the earlier application(s) in accordance with Art. 76 EPC: 24827108.2 / 4 637 884
(71) Applicant: Shaheen Innovations Holding Limited, Abu Dhabi (AE)
(72) Inventor: ALSHAIBA SALEH GHANNAM ALMAZROUEI, Mohammed, Al Maryah Island (AE); LAMOUREUX, Clement, Al Maryah Island (AE); MACHOVEC, Jeffrey, Al Maryah Island (AE); DIVAKARAN, Dinil, Al Maryah Island (AE); MOHAMED HUSSEIN ARBAB, Ahmed, Al Maryah Island (AE)
(74) Representative: Forresters IP LLP

(57) **Abstract**

A PCB for a driver for use with a mist generator cartridge, the PCB comprising a first portion carrying electronic components; a second portion configured to connect electrically to the mist generator cartridge; and a third portion positioned between the first portion and the second portion, the third portion including conducting tracks providing an electrical connection between the first portion and the second portion. The first portion lies in a first plane, and the second portion lies in a second plane, the second plane being transverse to the first plane. The drive signal connectors each extend from the first portion such that a longitudinal axis of each drive signal connector is parallel to the first plane, such that when the mist generator cartridge is coupled to the driver, the drive signals and the control signals are communicated from the driver to the mist generator cartridge to drive and control the operation of the mist generator cartridge.

## Description

### FIELD

The present invention relates to a driver for a mist generator. The present invention more particularly relates to a driver for a mist generator which atomises a liquid by ultrasonic vibrations.

### BACKGROUND

Mist generators, also known as electronic vaporising inhalers or vape devices, are used for generating a mist or vapour for inhalation by a user. The mist may contain nicotine or a therapeutic, drug or medicine which is inhaled by a user and absorbed into the user's blood stream.

Many conventional mist generators are supplied as a single-use device, which is designed to be disposed of after liquid within the device is depleted or charge in the battery of the device is depleted. The disposal of such devices significantly contributes to e-waste and damages the environment. Further, various valuable materials are used in the production of mist generators and the lithium-ion batteries within the mist generators and these valuable materials are often not recovered when the device is discarded.

Some conventional mist generators attempt to overcome the environmental issues by providing a reusable driver which releasably attaches to a mist generator cartridge or pod containing. The driver typically contains a battery and electronic components which control the operation of the mist generator cartridge. An electrical connection needs to be established between the driver and the mist generator cartridge so the driver can provide drive signals to activate and deactivate the mist generator cartridge. The electrical connection is typically formed by electrical contacts on the mist generator cartridge which engage respective electrical contacts on the driver.

Attaching and detaching the mist generator cartridge from the driver requires a mechanical interaction between the mist generator cartridge and the driver and mechanical wear inevitably occurs at any contact surfaces, including at the electrical contacts on the mist generator cartridge and the driver. Over time, the mechanical wear degrades the electrical contacts and can cause the electrical connection between the mist generator cartridge and the driver to become unreliable or to cease to function entirely.

A need exists in the art for an improved driver for a mist inhaler which seeks to address at least some of the problems described herein.

### BRIEF DESCRIPTION OF THE INVENTION

The present invention provides a PCB as claimed in claim 1, and a driver as claimed in claim 6. The present invention also provides preferred embodiments as claimed in the dependent claims.

The various examples of this disclosure which are described below have multiple benefits and advantages over conventional mist generators. Some of the benefits and advantages are set out in the description below.

A driver of some examples of this disclosure comprises printed circuit boards (PCBs) that are connected to one another by at least one flexible electrical connector. This enables the location and orientation of the electrical connections on the driver, which connect electrically to electrical contacts provided on a mist inhaler cartridge, to be set to improve the durability and reliability of the electrical connections. The improved durability helps to minimise e-waste and its associated damage to the environment.

A further benefit of the configuration of the driver is that the configuration minimises the overall size of the mist generator, making the driver and the mist generator easier to store and transport.

The configuration of a driver of a mist inhaler of some examples of this disclosure facilitates assembly and enables the driver to be assembled using a fully automated assembly line.

### REPRESENTATIVE FEATURES

Representative features are set out in the following clauses, which stand alone or may be combined, in any combination, with one or more features disclosed in the text and/or drawings of the specification.
1. A driver for use with a mist generator cartridge, the driver comprising:
   a driver casing incorporating a casing recess and an open end;
   a main printed circuit board (PCB) carrying electronic components configured to generate control signals to control the operation of the mist generator cartridge and drive signals to drive the mist generator cartridge to generate a mist, the main PCB being provided with a plurality of drive signal connectors each configured to connect electrically to a respective drive signal contact provided on the mist generator cartridge to communicate the drive signals to the mist generator cartridge;
   a connector PCB carrying a plurality of control signal connectors each configured to connect electrically to a respective one of a plurality of control signal contacts provided on the mist generator cartridge;
   at least one flexible electrical connector coupled between the main PCB and the connector PCB, wherein the at least one flexible electrical connector provides an electrical connection between the main PCB and the connector PCB to communicate the control signals between the main PCB and the connector PCB; and
   a driver shell carrying the main PCB in a first plane and the connector PCB in a second plane with the at least one flexible electrical connector being flexed so that a first portion of the at least one flexible electrical connector is in the first plane and a second portion of the at least one flexible electrical connector is in the second plane, the second plane being transverse to the first plane,
   wherein the driver shell is positioned at least partly within the casing recess, and
   wherein the drive signal connectors each extend from the main PCB in a plane parallel to the first plane and the control signal connectors each extend from the connector PCB in a plane parallel to the first plane, such that when the mist generator cartridge is coupled to the driver with part of the mist generator cartridge extending into the driver casing through the open end, the drive signal connectors connect electrically to the drive signal contacts on the mist generator cartridge and the control signal connectors connect electrically to the control signal contacts on the mist generator cartridge so that the drive signals and the control signals are communicated from the driver to the mist generator cartridge to drive and control the operation of the mist generator cartridge.
2. The driver of clause 1, wherein the wherein the drive signal connectors are positioned on a first side of the first plane and the control signal connectors are positioned on a second side of the first plane
3. The driver of clause 1 or clause 2, wherein the control signal connectors and the drive signal connectors are each elongate with a contact surface at one end, the driver shell carrying the main PCB and the connector PCB in a position relative to one another with the contact surfaces of the control signal connectors and the drive signal connectors terminating in a contact plane to align with the control signal contacts and drive signal contacts in a plane on the mist generator cartridge.
4. The driver of clause 3, wherein the driver comprises:
   a connector protector which is resiliently deformable and which is provided with a plurality of apertures, wherein one of the control signal connectors or the drive signal connectors extends through a respective one of the plurality of apertures with the contact surface of the control signal connector or the drive signal connector projecting outwardly from the connector protector.
5. The driver of clause 3 or clause 4, wherein the control signal connectors and the drive signal connectors are spring-loaded pin connectors.
6. The driver of clause 5, wherein each drive signal connector is electrically connected to a conductive track of the main PCB along at least part of a side of the drive signal connector with the side of the drive signal connector being parallel to a plane of the main PCB.
7. The driver of clause 5 or clause 6, wherein each control signal connector extends through a respective aperture in the connector PCB and a portion of the control signal connector remote from the contact surface is electrically connected to a conductive track of the connector PCB with the control signal connector being transverse to a plane of the connector PCB.
8. The driver of any one of the preceding clauses, wherein the driver shell comprises a body and a connector PCB holder, the connector PCB holder holding the connector PCB and being releasably attached to the body by an interference fit attachment.
9. The driver of clause 8, wherein the driver comprises:
   at least one magnet which is carried by the connector PCB holder, each magnet being positioned on the connector PCB holder to attract a metal portion of the mist generator cartridge when part of the mist generator cartridge extends into the driver casing through the open end.
10. The driver of any one of the preceding clauses, wherein the driver comprises:
   a further PCB;
   a further PCB holder for holding the further PCB in a position spaced apart from and at least partly superimposed on the main PCB and in a plane parallel to the first plane; and
   a plurality of flexible electrical connectors, wherein a first one of the plurality of flexible electrical connectors is coupled between the main PCB and the further PCB and a second one of the plurality of flexible electrical connectors is coupled between the further PCB and the connector PCB.
11. The driver of clause 10, wherein the further PCB carries a light emitting diode (LED) and the further PCB holder holds the further PCB in a position in which the LED is at least partly aligned with a window in the driver casing so that light emitted from the LED is visible to a user through the window in the driver casing.
12. The driver of clause 10 or clause 11, wherein the driver comprises:
   a further flexible electrical connector; and
   a temperature sensor coupled electrically to a first portion of the further flexible electrical connector, a second portion of the further flexible electrical connector being coupled to the further PCB, the further flexible electrical connector providing an electrical connection between the temperature sensor and the further PCB and the temperature sensor being spaced apart from the further PCB along part of a length of the further flexible electrical connector and superimposed on a battery of the driver to sense the temperature of the battery, wherein the temperature sensor provides a temperature signal which is communicated from the temperature sensor to the main PCB by the further flexible electrical connector, the further PCB and the first one of the plurality of flexible electrical connectors.
13. The driver of any one of the preceding clauses, wherein each flexible electrical connector is a flexible PCB.
14. The driver of clause 13, wherein each flexible PCB comprises a conductive track that is formed integrally with a conductive track of at least one of the main PCB, the connector PCB and a further PCB.
15. The driver of any one of the preceding clauses, wherein the driver comprises:
   an airflow sensor which is mounted on the main PCB; and
   an airflow sensor port which is coupled to the airflow sensor and which provides an airflow passage from the airflow sensor to an airflow connector, wherein the airflow connector comprises an aperture that is in a plane parallel to the second plane and positioned to connect to an airflow channel on the mist generator cartridge when the mist generator cartridge is coupled to the driver.
16. The driver of any one of the preceding clauses, wherein the driver casing comprises a further open end which is at least partly closed by a portion of the driver shell.
17. A mist generator for generating a mist for inhalation by a user, the mist generator comprising:
   a driver according to any one of the preceding clauses;
   a mist generator cartridge coupled to the driver, the mist generator cartridge incorporating:
      a plurality of drive signal contacts each connected electrically to a respective one of the drive signal connectors to receive the drive signals from the driver to drive the mist generator cartridge to generate a mist; and
      a plurality of control signal contacts each connected electrically to a respective one of the control signal connectors to receive the control signals from the driver to control the operation of the mist generator cartridge.
18. The mist generator of clause 17, wherein the mist generator cartridge comprises:
   a liquid chamber containing a liquid to be atomised; and
   an ultrasonic transducer which is coupled electrically to the drive signal contacts to receive the drive signals from the driver, wherein the drive signals cause the ultrasonic transducer to vibrate the liquid to generate a mist.
19. A method of assembling a driver for use with a mist generator cartridge, the method comprising:
   providing a main printed circuit board (PCB), a connector PCB and at least one flexible electrical connector coupled between the main PCB and the connector PCB;
   positioning the main PCB, the connector PCB and the at least one flexible electrical connector in an assembly plane;
   mounting electronic components to the main PCB;
   mounting a plurality of drive signal connectors to the main PCB with the drive signal connectors each extending from the main PCB in a plane parallel to the assembly plane;
   mounting a plurality of control signal connectors to the connector PCB with the control signal connectors each extending from the connector PCB in a plane transverse to the assembly plane;
   positioning the main PCB at least partly in a driver shell so that the driver shell carries the main PCB in a first plane;
   moving the connector PCB relative to the main PCB and flexing the at least one flexible electrical connector and positioning the connector PCB at least partly in the driver shell so that the driver shell carries the connector PCB in a second plane with the at least one flexible electrical connector being flexed so that a first portion of the at least one flexible electrical connector is in the first plane and a second portion of the at least one flexible electrical connector is in the second plane, the second plane being transverse to the first plane; and
   positioning a part of the driver shell in a casing recess of a driver casing so that the driver casing at least partly surrounds the part of the driver shell and at least part of the main PCB carried by the driver shell.
20. The method of clause 19, wherein the method is performed using an automated assembly line.

### BRIEF DESCRIPTION OF THE FIGURES

In order that the present disclosure may be more readily understood, preferable embodiments thereof will now be described, by way of example only, with reference to the accompanying drawings, in which:
Figure 1 is a diagrammatic perspective view of a mist generator of an example of this disclosure.
Figure 2 is a diagrammatic perspective view of a mist generator cartridge of an example of this disclosure.
Figure 3 is a diagrammatic perspective view of a driver of an example of this disclosure.
Figure 4 is a diagrammatic perspective view of part of a driver of an example of this disclosure.
Figure 5 is a diagrammatic exploded perspective view of a driver of an example of this disclosure.
Figure 6 is a diagrammatic perspective view of part of a driver of an example of this disclosure.
Figure 7 is a diagrammatic perspective view of part of a driver of an example of this disclosure.
Figure 8 is a diagrammatic perspective view of part of a driver of an example of this disclosure.
Figure 9 is a diagrammatic perspective view of part of a driver of an example of this disclosure.
Figure 10 is a diagrammatic perspective view of part of a driver of an example of this disclosure.
Figure 11 is a diagrammatic perspective view of part of a driver of an example of this disclosure.
Figure 12 is a diagrammatic perspective view of part of a driver of an example of this disclosure.
Figure 13 is a diagrammatic perspective view of part of a driver of an example of this disclosure.
Figure 14 is a diagrammatic perspective view of part of a driver of an example of this disclosure.
Figure 15 is a diagrammatic perspective view of part of a driver of an example of this disclosure.
Figure 16 is a diagrammatic perspective view of a mist generator cartridge of an example of this disclosure.
Figure 17 is a diagrammatic exploded perspective view of a mist generator cartridge of an example of this disclosure.
Figure 18 is a diagrammatic perspective view of a mist generator cartridge of an example of this disclosure.
Figure 19 is a diagrammatic perspective cross-sectional view of part of a mist generator cartridge of an example of this disclosure.
Figure 20 is a diagrammatic exploded perspective view of a mist generator cartridge of an example of this disclosure.
Figure 21 is a diagrammatic perspective cross-sectional view of part of a mist generator cartridge of an example of this disclosure.
Figure 22 is a diagrammatic perspective view of a mist generator cartridge of an example of this disclosure.
Figure 23 is a schematic diagram of an integrated circuit arrangement of this disclosure.

### DETAILED DESCRIPTION OF THE DISCLOSURE

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.

The following disclosure provides many different embodiments, or examples, for implementing different features of the provided subject matter. Specific examples of components, concentrations, applications and arrangements are described below to simplify the present disclosure. These are, of course, merely examples and are not intended to be limiting. For example, the attachment of a first feature and a second feature in the description that follows may include embodiments in which the first feature and the second feature are attached in direct contact, and may also include embodiments in which additional features may be positioned between the first feature and the second feature, such that the first feature and the second feature may not be in direct contact. In addition, the present disclosure may repeat reference numerals and/or letters in the various examples. This repetition is for the purpose of simplicity and clarity and does not in itself dictate a relationship between the various embodiments and/or configurations discussed.

The following disclosure describes representative examples. Each example may be considered to be an embodiment and any reference to an "example" may be changed to "embodiment" in the present disclosure.

Ultrasonic mist generators are either disposable or reusable. The term "reusable" as used herein implies that a battery within the mist generator is rechargeable or replaceable or that the liquid within the mist generator is able to be replenished either through refilling or through replacement of a liquid tank. Preferably, the mist generator is reusable because both the battery is rechargeable and the liquid can be replenished.

In some examples, a mist generator cartridge (also known as a capsule or pod) containing a liquid (or e-liquid) and a vaporising unit is releasably attached to a driver. The driver contains a battery and electronic components which control the operation of the mist generator cartridge. An electrical connection is provided between the driver and the mist generator cartridge and there is mechanical contact between the driver and the mist generator cartridge.

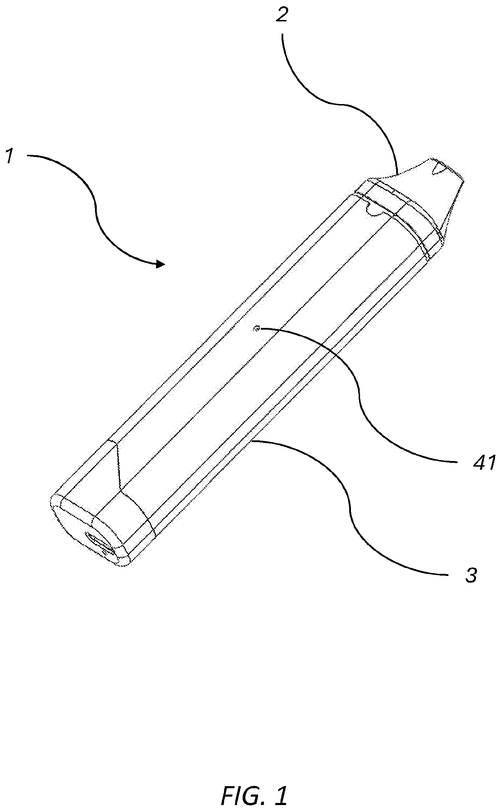

Referring initially to Figures 1 to 3 of the accompanying drawings, a mist generator 1 of one example of this disclosure comprises a mist generator cartridge 2 and a driver 3. In this example, the mist generator cartridge 2 is releasably attached to the driver 3 so that the mist generator cartridge 2. In other examples, the mist generator cartridge 2 is not releasably attached to the driver 3.

In some examples, the mist generator is for generating a mist containing nicotine for inhalation by a user. In other examples, the mist generator is for generating a mist containing a therapeutic or a medical drug for inhalation by a user.

In some examples, the mist generator cartridge 2 is configured to generate a mist by vibrating a liquid using an ultrasonic transducer to aerosolise a liquid and generate a mist. In these examples, the mist generator cartridge 2 comprises a liquid chamber containing a liquid to be atomised, an ultrasonic transducer and preferably also a capillary to carry the liquid from the liquid chamber to the surface of the ultrasonic transducer.

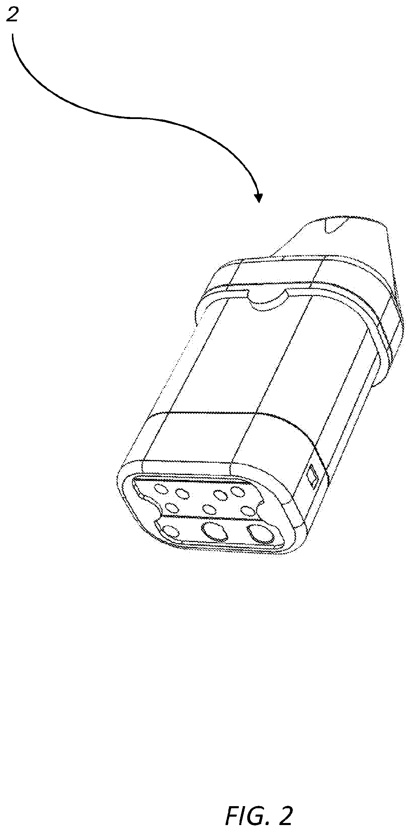

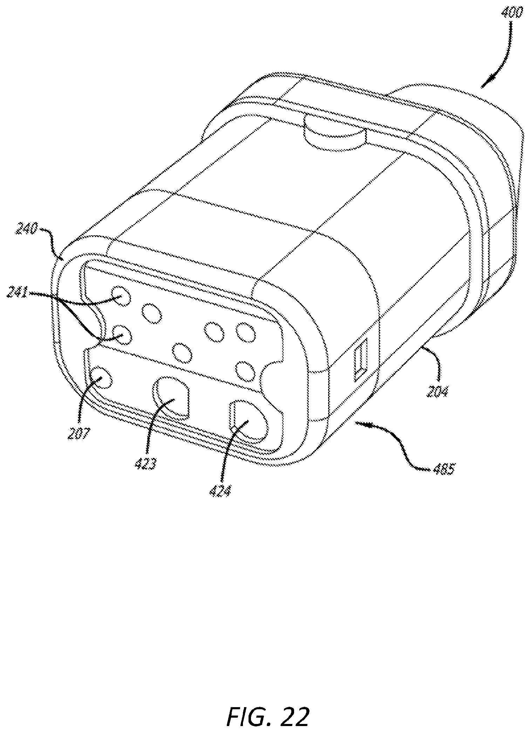

An example of a mist generator cartridge 2 for use with the driver 3 is shown in Figures 2 and 16 to 22. However, it is to be appreciated that the driver 3 may be used with other mist generator cartridges that may comprise only some of the elements of the mist generator cartridges described herein.

Examples of mist generator cartridges that may be used with the driver 3 of examples of this disclosure are disclosed in U.S. patent application no. 18/528,299, which is incorporated herein by reference in its entirety. However, it is to be appreciated that the driver 3 may be used with other mist generator cartridges.

In some examples, the driver 3 is for use with a mist generator cartridge that does not use ultrasound to generate a mist. In these examples, the mist generator cartridge comprises an atomisation unit that aerosolises a liquid to generate a mist using a different action, for instance using a heating element.

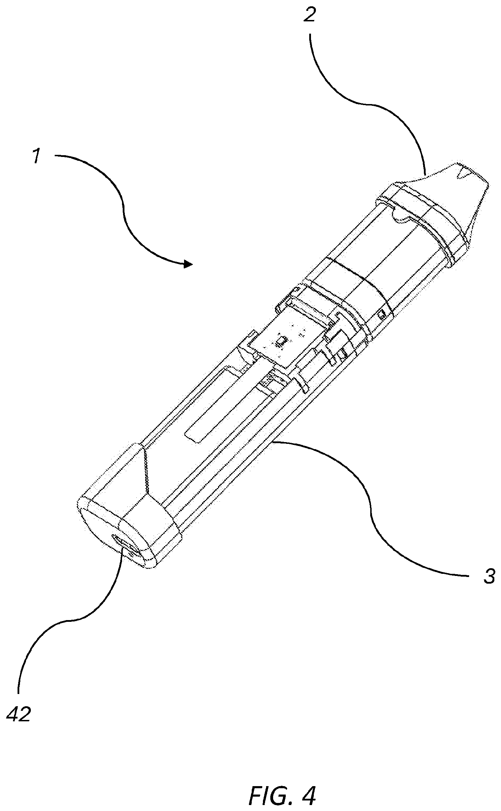

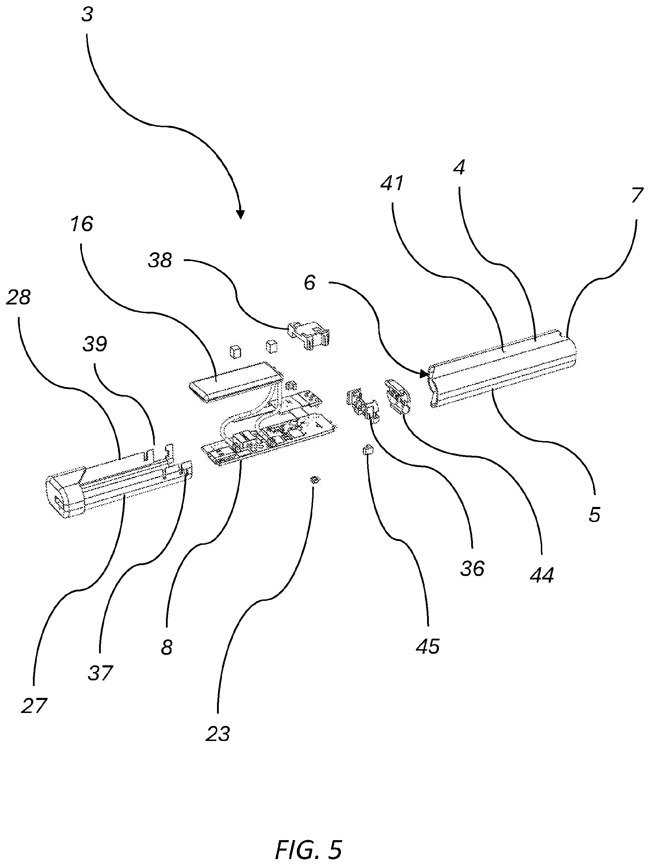

Referring now to Figures 4 to 8 of the accompanying drawings, the driver 3 comprises a driver casing 4. The driver casing 4 of this example comprises a generally tubular body 5 having a substantially square cross section. The driver casing 4 incorporates a casing recess 6 and an open end 7. In some examples, the driver casing 4 may comprise a tapered section along which the cross sectional area of the tubular body 5 reduces in size. In other examples, the tubular body 5 may have a different cross section shape.

The driver 3 further comprises a plurality of printed circuit boards (PCBs) which carry components of the driver 3. Each PCB, also known as a printed wiring board, is preferably in the form of a laminated structure of conductive and insulating layers. Each conductive layer comprises a pattern of traces, planes and other conductive portions that provide electrical connections between components mounted to the PCB. Each PCB may be rigid or semi-rigid. Each PCB is preferably a multi-layer PCB but in other examples at least one of the PCBs may be a single-layer PCB.

In this example of the disclosure, the plurality of PCBs comprises a main PCB 8, a connector PCB 9 and a further PCB 10. In other examples of the disclosure, the further PCB 10 may be omitted.

The main PCB carries electronic components configured to generate control signals to control the operation of the mist generator cartridge 2 and drive signals to drive the mist generator cartridge 2 to generate a mist.

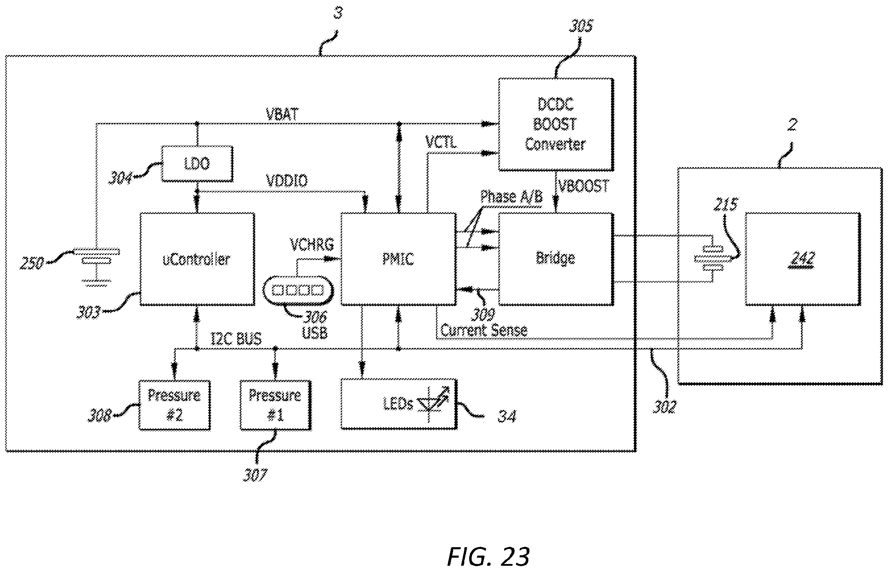

Some examples of electronic components that may be provided within the driver 3 are shown in Figure 23. However, it is to be appreciated that the driver 3 may incorporate other electronic components or may be configured differently from the examples described herein.

Examples of electronic components that may be provided within the driver 3 of examples of this disclosure are disclosed in U.S. patent application no. 18/528,299, which is incorporated herein by reference in its entirety.

In this example, the main PCB 8 and the further PCB 10 are coupled by a first flexible electrical connector 11 which provides an electrical connection between the main PCB 8 and the further PCB 10 to communicate control signals between the main PCB 8 and the further PCB 10.

In this example, the further PCB 10 and the connector PCB 9 are coupled by a second flexible electrical connector 12 which provides an electrical connection between the further PCB 10 and the connector PCB 9 to communicate control signals between the further PCB 10 and the connector PCB 9.

In other examples, the further PCB 10 and the second flexible electrical connector 12 may be omitted. In these examples, the main PCB 8 and the connector PCB 9 are coupled to one another by at least the first flexible electrical connector 11 which provides an electrical connection between the main PCB 8 and the connector PCB 9 to communicate control signals between the main PCB 8 and the connector PCB 9.

In examples of this disclosure, the flexible electrical connectors may be flexible PCBs. Each flexible PCB may comprise a conductive track that is formed integrally with a conductive track of at least one of the main PCB 8, the connector PCB 9 and the further PCB 10. In other examples, the flexible electrical connectors are another type of flexible electrical connector, such as a ribbon cable or a plurality of individual wires or cables.

In this example, the connector PCB 9 carries a plurality of control signal connectors 13 each configured to connect electrically to a respective control signal contact 241 provided on the mist generator cartridge 2 to communicate control signals to the mist generator cartridge 2. In this example, the main PCB 8 carries a plurality of drive signal connectors 14 each configured to connect electrically to a respective drive signal contact 423, 424 provided on the mist generator cartridge 2 to communicate drive signals to the mist generator cartridge 2.

In this example, the control signal connectors 13 and the drive signal connectors 14 are spring-loaded pin connectors or pogo pin connectors. In examples of this disclosure, the control signal connectors 13 and the drive signal connectors 14 are each elongate with a contact surface 15 at one end. In this example, the control signal connectors 13 are pogo pins having a sealed base.

The drive signal connectors 14 are configured to deliver the drive signals at a current of up to 3A, or more typically at up to 2A. The drive signals are preferably AC drive signals at a frequency of 0.5 MHz to 1.5 MHz, 2.8 MHz to 3.2 MHz or 3 MHz to 5 MHz depending on the application and required properties of the mist to be output from the mist generator.

In some examples, the further PCB 10 and the connector PCB 9 provide passthrough electrical connections that provide an electrical connection between the control signal connectors 13 and the main PCB 8. This enables the electronic components, such as integrated circuits, on the main PCB 8 to receive signals from and send signals to the mist generator cartridge via the control signal connectors 13.

In some examples described herein, the mist generator cartridge 2 comprises a one time programmable (OTP) IC that is configured to receive and transmit control signals from and to the main PCB of the driver 3 via the control signal connectors 13 when the mist generator cartridge 2 is coupled to the driver 3. For example, the electronic components on the main PCB 8 perform a handshake sequence with the OTP IC to authenticate the mist generator cartridge 2 for use with the driver 3.

In these and other examples, the electronic components on the main PCB 8 communicate control signals with the OTP IC using an I²C data bus. The control signals sent between the driver 3 and the mist generator cartridge 2 control the operation of the mist generator cartridge 2. The I²C bus utilises four of the five control signal connectors 13 to transmit and receive data between the OTP IC and the main PCB 8. The fifth control signal connector 13 is used as a detect pin to enable the driver 3 to detect when the mist generator cartridge 2 is connected to the driver 3.

In this example, the drive signal connectors 14 are mounted to the main PCB 8 and positioned such that a longitudinal axis of each drive signal connector 14 is parallel or generally parallel with a plane of the main PCB 8. In this example, each drive signal connector 14 is electrically connected to a conductive track of the main PCB 8 along at least part of a side of the drive signal connector 14 with the side of the drive signal connector 14 being parallel or generally parallel to a plane of the main PCB 8.

In this example, the control signal connectors 13 are positioned such that a longitudinal axis of the control signal connectors 13 is transverse or generally perpendicular or orthogonal to a plane of the connector PCB 9. In this example, each control signal connector 13 extends through a respective aperture in the connector PCB 9 and a portion of the control signal connector 13 remote from a contact surface of the control signal connector 13 is electrically connected to a conductive track of the connector PCB 9 with the control signal connector 13 being transverse, perpendicular or generally perpendicular to a plane of the connector PCB 9.

The driver 3 further comprises a battery 16 and a battery power connector 17 which is attached to the battery 16. In this example, the battery 16 is superimposed on the main PCB 8. The width of the battery 16 is preferably less than or equal to the width of the main PCB 8 and the length of the battery 16 is preferably less than or equal to the length of the main PCB 8. In this example, the battery power connector 17 is formed of two wires and electrically connects the battery 16 to the main PCB 8. The battery power connector 17 is secured to the main PCB 8 by a least one cap connector 18.

In this example, a third (further) flexible electrical connector 19 having an elongate body is coupled to the further PCB 10. In other examples, the third flexible electrical connector 19 may be coupled to the main PCB 8 or the connector PCB 9. In this example, the third flexible electrical connector 19 comprises a first portion 20 which is electrically coupled to a temperature sensor 21 and a further portion 22 which is electrically coupled to the further PCB 10. However, in other examples, the third flexible electrical connector 13 and the temperature sensor 21 are omitted entirely.

In this example, the device further comprises an airflow sensor 23 which is mounted on the main PCB 8. An airflow sensor port 24 is coupled to the airflow sensor 23 and provides an airflow passage from the airflow sensor 23 to an airflow connector 25. The airflow connector 25 comprises an aperture 26 that is in a plane parallel or generally parallel to the (second) plane of the connector PCB 9 and positioned to connect to an airflow channel 207 on the mist generator cartridge 2 when the mist generator cartridge 2 is coupled to the driver 3. The airflow sensor 23 senses a change in the pressure in an aerosol chamber in the mist generator cartridge 2 to sense when a user is drawing on the mist inhaler cartridge 2.

In other examples, the airflow sensor 23 may be mounted on the connector PCB 9, the further PCB 10, or omitted entirely. In some examples, the airflow sensor 23 may be mounted to the main PCB 8, the connector PCB 9 or the further PCB 10 by an interference fit.

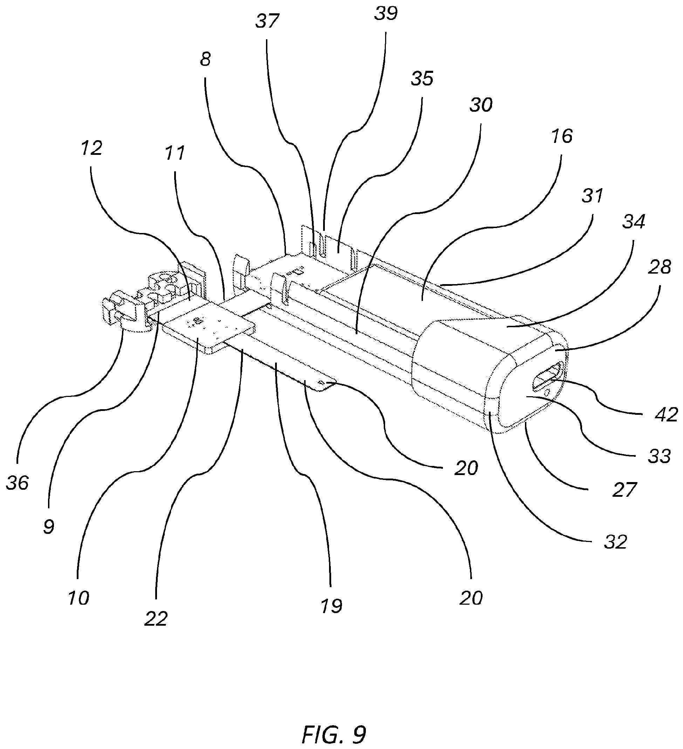

Referring now to Figures 9 to 12, the driver 3 further comprises a driver shell 27 which in this example is configured to carry the main PCB 8, the connector PCB 9, the further PCB 10 and the battery 16. The driver shell is positioned at least partly within the casing recess 6. A portion of the driver shell 27 closes a further open end of the driver casing 4. The driver shell 27 of this example may be attached to driver casing 4 by an attachment feature such that the driver shell 27 is enclosed at least partly by the driver casing 4 and the driver shell 27 does not move relative to the driver casing 4.

In this example of the disclosure, the driver shell 27 comprises a body 28 which is elongate. In this example, the body 28 has a substantially square cross section. The body 28 of this example is formed by a first side 29, a second side 30 and a third side 31 which are connected along elongate edges at substantially orthogonal angles to form a U-shape cross section. At a first end 32 of the body 28 a fourth side 33 is orthogonal to and connects the first side 29, second side 30 and third side 31 along short edges. In this example a fifth side 34, which is parallel to and offset from the first side 29 and connects the second side 30 and the third side 31, extends at least partially from the first end 32 of the body 28 along a longitudinal axis of the body 28. In this example, the first side 29, the second side 30, the third side 31, the fourth side 33 and the fifth side 34 are all substantially planar.

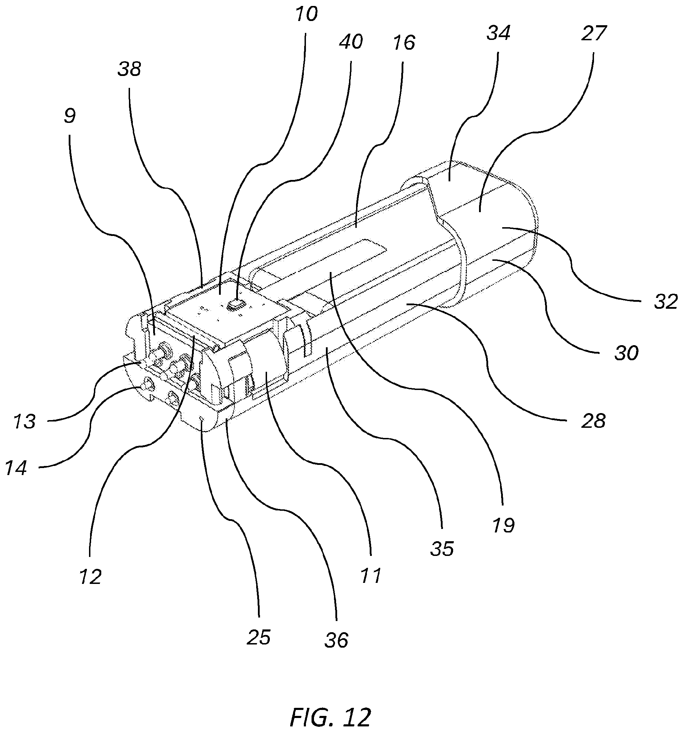

In the example of the disclosure shown in Figure 12, the driver shell 27 is configured to carry to the main PCB 8 in a first plane and the connector PCB 9 in a second plane. The second plane is transverse to the first plane. The second plane is preferably perpendicular or generally perpendicular to the first plane.

The driver shell 27 of this example comprises a body 35 and a connector PCB holder 36 which is configured to carry the connector PCB 9. The connector PCB holder 36 is releasably attached to the body 35 by an interference fit attachment. In this example, the connector PCB holder 36 is releasably attached to the body 35 by a connector attachment 37 provided on the third side 31. In this example, the connector attachment 37 comprises an aperture configured to receive a male component of a snap fit fitting provided on the connector PCB holder 36.

The driver shell 27 of this example further comprises a further PCB holder 38 which is configured to carry the further PCB 10 and attach to further attachments 39 provided on the second side 30 and the third side 31. The further PCB holder 38 holds the further PCB 10 in a third plane, which is preferably parallel or generally parallel to the first plane and spaced apart from the main PCB 8. In this example, the further attachments 39 comprise a plurality of grooves each configured to receive a rail provided on the further PCB holder 38. In other examples of the disclosure, the further PCB holder 38 may be omitted.

In the example shown in Figure 12, when the connector PCB holder 36 carries the connector PCB 9 and is attached to the connector attachments 37, the first flexible electrical connector 11 is flexed (or deformed elastically) so that a first portion of the first flexible electrical connector 11 is in the first plane and a second portion of the first flexible electrical connector 11 is in the second plane.

The driver shell 27 carries the main PCB 8 and the connector PCB 9 in a position relative to one another with the contact surfaces of the control signal connectors 13 and the drive signal connectors 14 terminating in a contact plane to align with the control signal contacts 241 and the drive signal contacts 423, 424 in a plane on the mist generator cartridge 2.

The contact plane is transverse, perpendicular or generally perpendicular to a plane of the main PCB 8 and the contact plane faces outwardly from one end of the driver 3. Consequently, when the mist generator cartridge 2 is inserted into the end of the driver casing 4, the control signal contacts and the drive signal contacts are moved in a plane parallel to or generally parallel to the contact plane. The control signal contacts and the drive signal contacts then come into contact with the control signal connectors 13 and the drive signal connectors 14 at substantially the same time as one another. Moving the control signal contacts and the drive signal contacts directly towards and onto the contact surfaces of the control signal connectors 13 and the drive signal connectors 14 avoids the need to slide the contact surfaces against the control signal contacts and the drive signal contacts. This minimises mechanical wear to the contact surfaces and the control signal contacts and the drive signal contacts. Consequently, the longevity of the driver 3 and the mist generator cartridge 2 are improved since the contact surfaces and the control signal contacts and the drive signal contacts can remain intact for longer with minimal mechanical wear.

There are also benefits to positioning the drive signal connectors 14 on a lower, first, side of the main PCB 8 and the control signal connectors 13 above an opposite upper, second, side of the main PCB 8. One such benefit is that the driver 3 is compact in size because the drive signal connectors 14 and the control signal connectors 13 can align approximately centrally with the respective contacts on the mist generator cartridge 2.

Another benefit is that relatively thick electrical tracks of the main PCB 8 are connected directly to the side of the drive signal connectors 14 to maximise conductivity and allow for a relatively high current of up to 3A to flow to the drive signal connectors 14. On the other hand, only a smaller (mA) current is required for the control signals to be communicated to the control signal connectors 13 and this smaller current can be conducted through the flexible electrical connectors 11, 12 which can benefit from the improved physical placement enabled by the flexing of the flexible electrical connectors 11, 12.

The drive signal connectors 14 each extend from the main PCB 8 in a plane parallel to or generally parallel to the first plane and the control signal connectors 13 each extend from the connector PCB 9 in a plane parallel to or generally parallel to the first plane. Consequently, when the mist generator cartridge 2 is coupled to the driver 3 with part of the mist generator cartridge 2 extending into the driver casing 4 through the open end, the drive signal connectors 14 connect electrically to the drive signal contacts 423, 424 on the mist generator cartridge 2 and the control signal connectors 13 connect electrically to the control signal contacts 241 on the mist generator cartridge 2. The drive signals and the control signals can therefore be communicated from the driver 3 to the mist generator cartridge 2 to drive and control the operation of the mist generator cartridge 2.

In some examples, the drive signal connectors 14 are positioned on a first side of the first plane and the control signal connectors 13 are positioned on a second side of the first plane. In this configuration, the main PCB is positioned in the first plane between the drive signal connectors 14 and the control signal connectors 13 which minimises the overall size/height of the driver 3. This is helps to ensure that the device has a small form factor, which is desirable for portability.

In the example shown in Figure 12, when the further PCB holder 38 carries the further PCB 10 and is attached to the further attachments 39, the second flexible electrical connector 12 is flexed so the first portion of the second flexible electrical connector 12 is in the third plane and a second portion of the second flexible electrical connector 12 is in the second plane. In other examples, such as examples where the further PCB 10 and the second flexible electrical connector 12 are omitted, the first flexible electrical connector 11 may be flexed so that a first portion of the first flexible electrical connector 11 is in the first plane and a second portion of the first flexible electrical connector 11 is in the second plane.

In this example, the further PCB 10 carries a light emitting diode (LED) 40, which may be used to notify a user of the operating state of the driver 3. In this example, the further PCB holder 38 holds the further PCB 10 in a position in which the LED 40 is at least partly aligned with a window 41 of the driver casing 4 so that light emitted from the LED 40 is visible to a user through the window 41 of the driver casing 4.

Providing the LED 40 on the further PCB 10 simplifies the method of assembling the driver 3 by enabling the LED 40 to be positioned and aligned with the window 41 in the driver casing 4 by moving the second flexible electrical connector 11. This avoids the need for a separate, typically manual, step in the manufacturing process to mount the LED 40 in position on the driver casing 4. The configuration therefore helps ensure that the driver 3 can be assembled using an automated or, preferably, a fully automated assembly line.

In this example, the fourth side comprises an aperture 42 which is configured to provide access to an electrical interface 43 provided on the main PCB 8.

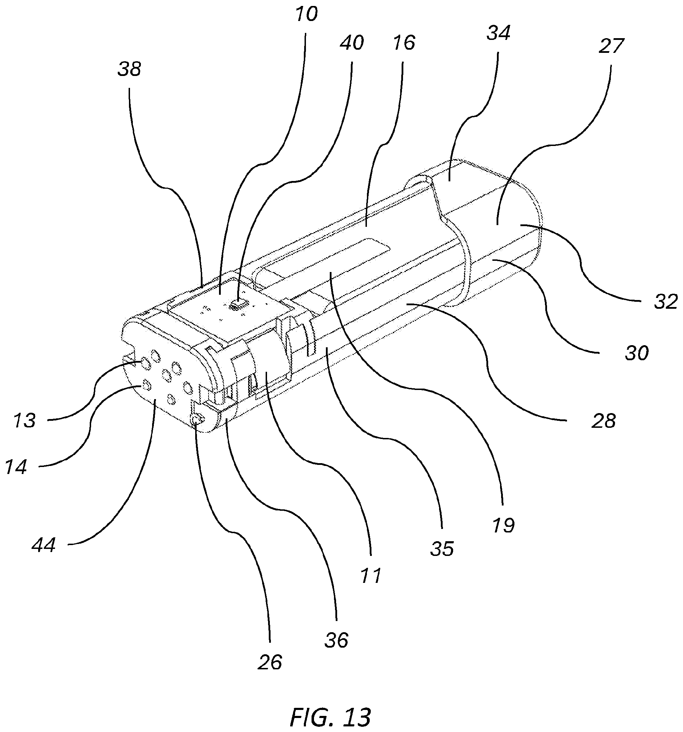

Turning now to Figure 13, the driver 3 of this example further comprises a resiliently deformable connector protector 44 comprising a plurality of apertures 45. The connector protector 44 is positioned such that one of the control signal connectors 13 or the drive signal connectors 14 extends through a respective one of the plurality of apertures 45 with a contact surface of the control signal connector 13 or the drive signal connector 14 projecting outwardly from the connector protector 44.

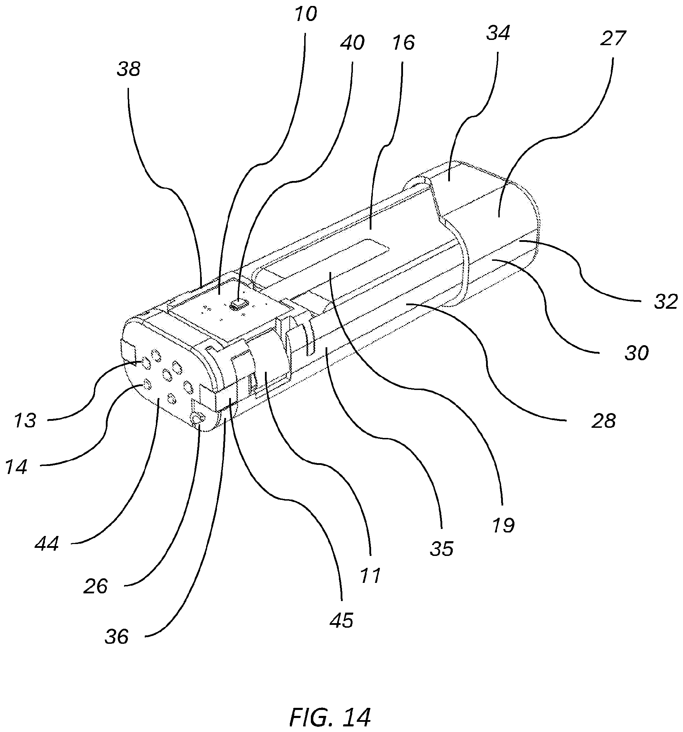

Turning now to Figure 14, the driver 3 of this example further comprises at least one magnet 46 which is carried by the connector PCB holder 36, each magnet 46 is positioned on the connector PCB holder 36 to attract a metal portion 485 of the mist generator cartridge 2 when part of the mist generator cartridge 2 extends into the driver casing 4 through the open end to couple the mist generator cartridge 2 to the driver 3. In some examples, the driver 3 comprises a detent, latch or other interference fit arrangement that restricts the movement of the mist generator cartridge 2 relative to the driver 3 to further minimise the risk of the mist generator cartridge 2 becoming detached accidentally from the driver 3.

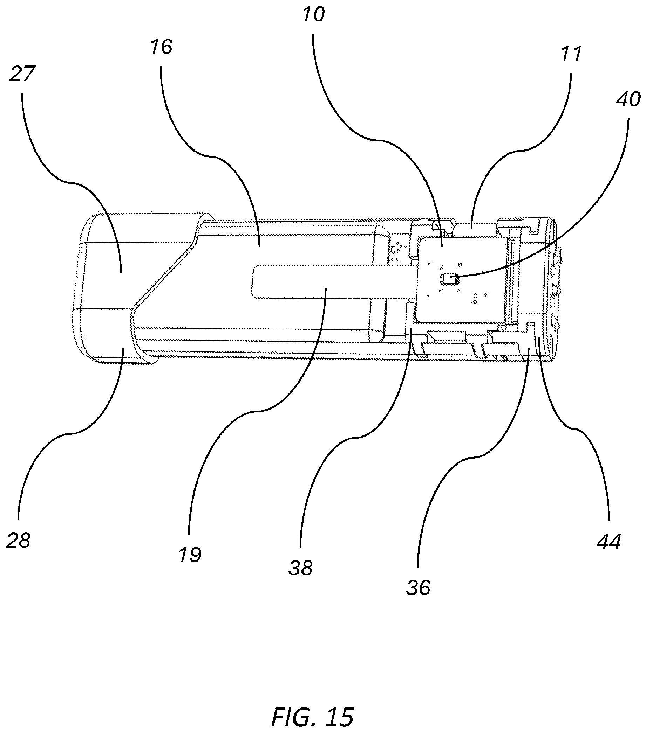

Turning now to Figure 15, in this example, the third flexible electrical connector 19 is positioned such that a longitudinal axis of the third flexible electrical connector 19 is parallel to the longitudinal axis of the main PCB 8. The temperature sensor 21 is spaced apart from the further PCB 10 along part of a length of the third flexible electrical connector 19 and superimposed on the battery 16 to sense the temperature of the battery 16. The temperature sensor 21 provides a temperature signal which is communicated from the temperature sensor 21 to the main PCB 8 by the further third electrical connector 17, the further PCB 10 and the first flexible electrical connector 11.

The main PCB 8 is provided with a charger circuit that controls the charging of the battery 16. The charger circuit is configured to control the charging of the battery 16 in response to the temperature signal from the temperature 21 to minimise or prevent the battery overheating during charging. The configuration of the temperature sensor 21 on the third flexible electrical connector 19 enables the temperature of the battery 21 to be measured accurately since the temperature sensor 21 is positioned toward the middle section of the battery 21.

Providing the temperature sensor 21 on the third flexible electrical connector 19 simplifies the method of assembling the driver 3 by enabling the temperature sensor 21 to be positioned correctly relative to the battery by moving the third flexible electrical connector 19. This avoids the need for a separate, typically manual, step in the manufacturing process to mount the temperature sensor 21 in position. The configuration therefore helps ensure that the driver 3 can be assembled using an automated or, preferably, a fully automated assembly line.

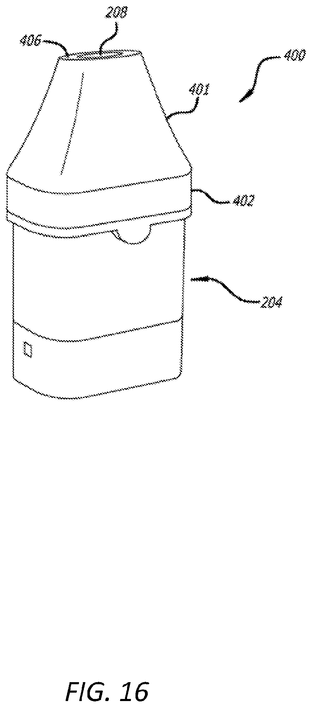

Referring now to Figures 16-22 of the accompanying drawings, the mist generator cartridge 2 described above may incorporate some or all of the elements of the mist generator cartridge 400 described below. The mist generator cartridge 400 is one example of a type of mist generator cartridge that may be used with the driver 3.

The mist generator cartridge 400 comprises a housing 204 comprising a liquid chamber 218 for containing a liquid to be atomised. The liquid chamber 218 may contain any e-liquid or liquid described in the present disclosure or any other liquid which is to be atomised.

The mist generator cartridge 400 comprises a mouthpiece 401 that is coupled to the housing 204. The mouthpiece 401 comprises a base 402 having an opening 403 which receives a connector portion 404 of the housing 204. The connector portion 404 comprises at least one latch element 405 that engages a latch recess (not shown) to retain the mouthpiece 401 in connection with the housing 204.

The mouthpiece 401 narrows progressively from the base 402 to a distal end 406. The distal end 406 comprises a mist outlet port 208 to enable mist to be output from the mist generator cartridge 400 for inhalation by a user.

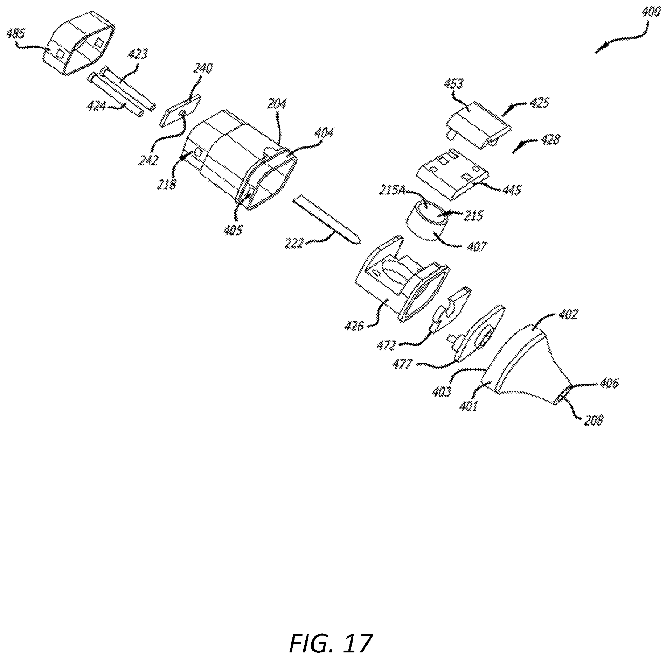

Referring now to Figure 17 of the accompanying drawings, the mist generator cartridge 400 comprises an ultrasonic transducer 215. In this example, the ultrasonic transducer 215 is received within an ultrasonic transducer stack 407 which may alternatively be known as an ultrasonic transducer assembly. While the ultrasonic transducer stack 407 is described and shown herein in use with the mist generator cartridge 400, it is to be appreciated that the ultrasonic transducer stack 407 may be provided in isolation and/or integrated for use with any other type of device that comprises an ultrasonic transducer.

The mist generator cartridge 400 comprises a sonication assembly 425 which receives the ultrasonic transducer stack 407. The sonication assembly 425 comprises a first assembly portion 426 comprising a recess 427 that receives and retains the ultrasonic transducer 215 within the ultrasonic transducer stack 407.

The sonication assembly 425 comprises a second assembly portion 428 which, when assembled, is coupled to the first assembly portion 426. As described below, the configuration of the first and second assembly portions 426, 428 enables the first and second assembly portions 426, 428 to fit together easily, which facilitates assembly of the mist generator cartridge 400. The parts of the sonication assembly 425 may be assembled using automated robots on a production line with minimal human intervention. The mist generator cartridge 400 is therefore configured to be mass produced on a production line relatively easily and at low cost compared with conventional mist generator cartridges.

At least one of the first assembly portion 426 and the second assembly portion 428 comprises a resiliently deformable section that forms a seal between the first assembly portion 426 and the second assembly portion 428. The seal minimises or prevents a fluid from leaking between the first assembly portion 426 and the second assembly portion 428. The resiliently deformable configuration of the first and second assembly portions 426, 428 avoids the need for a sealant or adhesive to be applied, which reduces the complexity and cost of manufacturing the mist generator cartridge 400.

In this example, both the first assembly portion 426 and the second assembly portion 428 are of a resiliently deformable material. In other examples, only one of the first assembly portion 426 and the second assembly portion 428 is of a resiliently deformable material.

In this example, the resiliently deformable material is silicone. In other examples, the resiliently deformable material is a different resiliently deformable plastic material.

The mist generator cartridge 400 minimises or eliminates the possibility of any liquid leaking out from within the mist generator cartridge 400. This is critical for consistency of dosage delivery. A leaking device may have less liquid (containing nicotine, drug, etc.) in the liquid chamber 218 than what would be prescribed, for instance as part of a nicotine cessation programme or drug/therapeutic delivery for treatment of a condition.

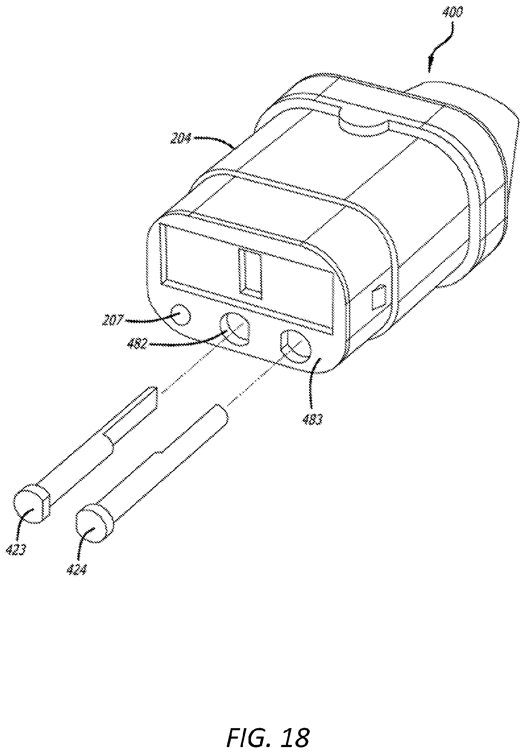

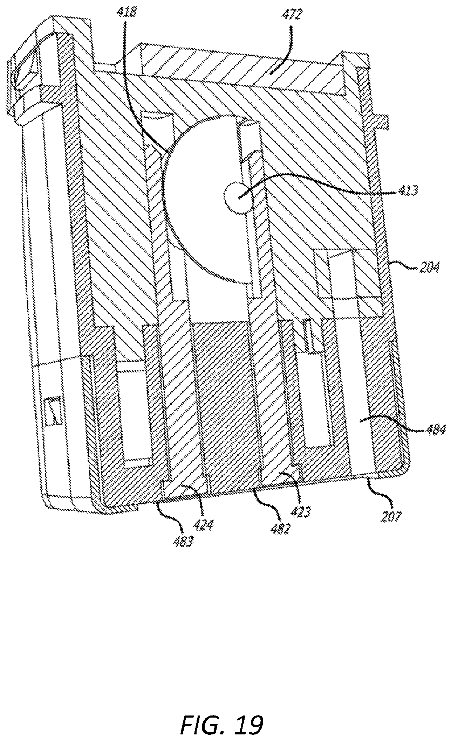

Referring now to Figures 18 and 19 of the accompanying drawings, the first and second elongate device terminals 423, 424 are inserted through respective terminal apertures 482, 483 in the base of the housing 204. The first elongate device terminal 423 extends through the mist generator cartridge 400 to connect electrically to the first electrical transducer contact 413. The second elongate device terminal 424 extends through part of the mist generator cartridge 400 to connect electrically to the shell 418. The configuration of the ultrasonic transducer stack 407 and the resiliently deformable material of the sonication assembly 425 ensures that no liquid can seep between the shell 418 and the sonication assembly 425. The first and second elongate device terminals 423, 424 are therefore isolated from the liquid within the mist generator cartridge 400 that might otherwise disrupt the AC drive signal delivered to the ultrasonic transducer 215 and cause the mist generator cartridge 400 to malfunction.

The first and second elongate device terminals 423, 424 provide electrical connections from the ends of the elongate device terminals 423, 424 that are accessible at the base of the housing 204 to the terminals of the ultrasonic transducer 215. An AC drive signal generated by the driver 3 may therefore be transmitted to the ultrasonic transducer 215 via the elongate device terminals 423, 424.

Also visible in Figures 18 and 19 is an airflow aperture 207 that is provided in the base of the housing 204. The airflow aperture 207 is coupled fluidly to an air channel 484 that extends through the mist generator cartridge 400 to the sonication assembly air inlet port 451.

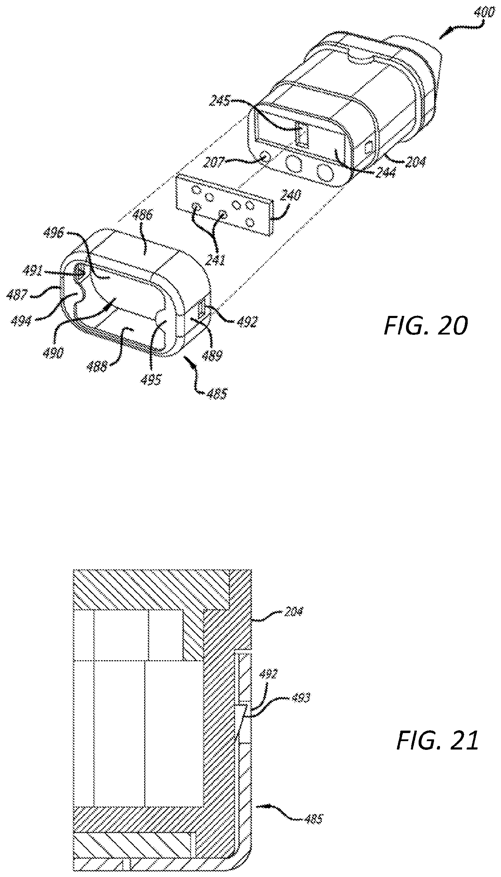

Referring now to Figures 20 and 21 of the accompanying drawings, the mist generator cartridge 400 comprises an end cap 485 that attaches to the lower end of the housing 204. The end cap 485 comprises four walls that define a recess 490 that receives an end portion of the housing 204. Opposing side walls 487, 489 each comprise a respective retainer slot 491, 492. Each retainer slot 491, 491 receives a respective outwardly directed chamfer 493 (only one of which is visible in Figures 53 and 54). Each chamfer 493 retains the end cap 485 attached to the housing 204.

The end cap 485 comprises two inwardly directed tabs 494, 495 at the base of the end cap 485. The tabs 494, 495 are superimposed on respective ends of the printed circuit board 240 when the end cap 485 is attached to the housing 204. The printed circuit board 240 carries a programmable or one time programmable integrated circuit or OTP IC 242. The tabs 494, 495 retain the printed circuit board 240 in position within the recess 244 on the housing 204.

The end cap 484 comprises an aperture 496 to allow access to the electrical contact 241 on the printed circuit board 240 and access to the elongate device terminals 423, 424. The aperture 496 also allows air to flow into and out from the airflow aperture 207 on the housing 204.

In this example, the end cap 485 is of metal and the end 485 enables the mist generator cartridge 400 to be retained in the recess 203 of the driver 3 by a magnetic force through attraction to a magnet 45 provided on the driver 3. However, in other examples, the end cap 485 may be of a different material or may be omitted entirely.

Referring now to Figure 23 of the accompanying drawings, in some examples the driver 3 comprises an ultrasonic transducer driver microchip which is referred to herein as a power management integrated circuit or PMIC 300. The PMIC 300 is a microchip for driving a resonant circuit. The resonant circuit is an inductance (L) capacitance (C) circuit (LC tank), an antenna or, in this case, a piezoelectric transducer (the ultrasonic transducer 215).

The driver 3 also comprises a second microchip which is referred to herein as a bridge integrated circuit or bridge IC 301 which is electrically connected to the PMIC 300. The bridge IC 301 is a microchip for driving a resonant circuit, such as an LC tank, an antenna or a piezoelectric transducer.

In this example, the PMIC 300 and the bridge IC 301 are mounted to the main PCB 8 of the driver 3. Many of the other electronic components of the driver 3 are also mounted to the main PCB 8.

When the mist generator cartridge 2, 400 is coupled to the driver 3, the OTP IC is electrically connected to the PMIC 300 to receive power from the PMIC 300 such that the PMIC 300 can manage the voltage supplied to the OTP IC 242. The OTP IC 242 is also connected to a communication bus 302 in the driver 3. In this example, the communication bus 302 is an I²C bus but in other examples the communication bus 302 is another type of digital serial communication bus.

The ultrasonic transducer 215 in the mist generator cartridge 2, 400 is electrically connected to the bridge IC 301 so that the ultrasonic transducer 215 may be driven by an AC drive signal generated by the bridge IC 301 when the mist generator is in use.

The driver 3 comprises a processor in the form of a microcontroller 303 which is electrically coupled for communication with the communication bus 302. In this example, the microcontroller 303 is a Bluetooth^{™} low energy (BLE) microcontroller. The microcontroller 303 receives power from a low dropout regulator (LDO) 304 which is driven by the battery 250. The LDO 304 provides a stable regulated voltage to the microcontroller 303 to enable the microcontroller 303 to operate consistently even when there is a variation in the voltage of the battery 250.

The driver 3 comprises a voltage regulator in the form of a DC-DC boost converter 305 which is powered by the battery 250. The boost converter 305 increases the voltage of the battery 250 to a programmable voltage VBOOST. The programmable voltage VBOOST is set by the boost converter 305 in response to a voltage control signal VCTL from the PMIC 300. As will be described in more detail below, the boost converter 305 outputs the voltage VBOOST to the bridge IC 301. In other examples, the voltage regulator is a buck converter or another type of voltage regulator which outputs a selectable voltage.

The voltage control signal VCTL is generated by a digital to analogue converter (DAC) which, in this example, is implemented within the PMIC 300. The DAC is not visible in Figure 23 since the DAC is integrated within the PMIC 300. The DAC and the technical benefits of integrating the DAC within the PMIC 300 are described in detail below.

In this example, the PMIC 300 is connected to a power source connector in the form of a universal serial bus (USB) connector 306 so that the PMIC 300 can receive a charging voltage VCHRG when the USB connector 306 is coupled to a USB charger.

In this example, the LED 40 of the driver 3 is controlled by the PMIC 300.

The microcontroller 303 functions as a master device on the communication bus 302, with the PMIC 300 being a first slave device, the OTP IC 242 being a second slave device, the second pressure sensor 308 being a third slave device and the first pressure sensor 307 being a fourth slave device. The communication bus 302 enables the microcontroller 303 to control the following functions within the driver 3:
1. All functions of the PMIC are highly configurable by the microcontroller 303.
2. The current flowing through the ultrasonic transducer 215 is sensed by a high bandwidth sense and rectifier circuit at a high common mode voltage (high side of the bridge). The sensed current is converted into a voltage proportional to the rms current and provided as a buffered voltage at a current sense output pin 309 of the bridge IC 301. This voltage is fed to and sampled in the PMIC 300 and made available as a digital representation via I²C requests. Sensing the current flowing through the ultrasonic transducer 215 forms part of the resonant frequency tracking functionality. As described herein, the ability of the device to enable this functionality within the bridge IC 301 provides significant technical benefits.
3. The DAC (not shown in Figure 23) integrated within the PMIC 300 enables the DC-DC boost converter voltage VBOOST to be programmed to be between 10V and 20V.
4. The microcontroller 303 enables the charger sub-system of the driver 3 to manage the charging of the battery 250, which in this example is a single cell battery.
5. A Light Emitting Diode (LED) driver module (not shown) is powered by the PMIC 300 to drive and dim the LED 40 either in linear mode or in gamma corrected mode.
6. The microcontroller 303 is able to read Pressure#1 value from the airflow sensor 23.

A method of assembling or manufacturing a driver 3 of examples of this disclosure will now be described.

In some examples, all or some of the PCBs 8-10 and the flexible electrical connectors 11, 12 are manufactured as a one component with continuous conducting tracks extending in a layer across each of the PCBs 8-10 and the flexible electrical connectors 11, 12. This initial step of manufacturing the PCBs 8-10 and the flexible electrical connectors 11, 12 as one component reduces the complexity of the manufacturing process since it removes the need to provide for additional soldering steps to attach the PCBs 8-10 and the flexible electrical connectors 11, 12 together. However, in other examples, at least some of the PCBs 8-10 are manufactured separately from one another and connected to one another by attaching the flexible electrical connectors 11, 12.

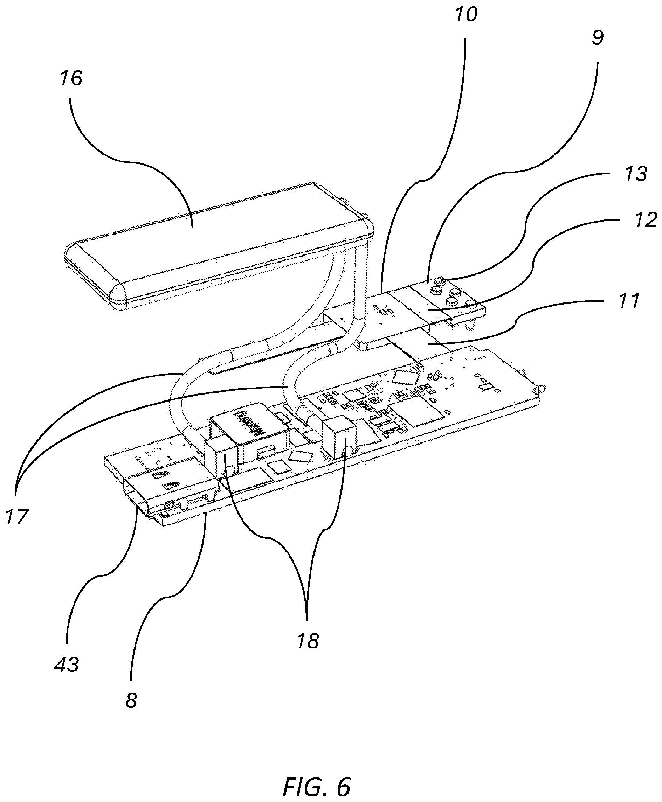

Once the PCBs 8-10 and the flexible electrical connectors 11, 12 are connected to one another (either initially as one component or by assembly), the PCBs 8-10 and the flexible electrical connectors 11, 12 are positioned or laid flat in an assembly plane, as shown in Figure 6. The electronic components are then placed on the main PCB 8, either on a first, upper surface, or on a lower, second, surface of the main PCB 8. The drive signal connectors 14 are placed in position against the lower, second, surface of the main PCB 8 on the opposite side of the main PCB 8 to the integrated circuits and other electronic components. The components and the drive signal connectors 14 are preferably placed using a pick and place machine or other autonomous robotic system. Other components, such as the LED 40 are also placed in position on the main PCB 8, the connector PCB 9 or the further PCB 10. The PCBs 8-10 are then heated to solder the components in position, for instance using a solder reflow process.

The control signal connectors 13 are inserted through the aperture in the connector PCB 9 and, in some examples, are soldered on one side of the connector PCB 9 by an additional soldering process.

The assembly process is facilitated by the configuration of the PCBs 8-10 and the flexible electrical connectors 11, 12 which enables them to be positioned or laid flat in the assembly plane on an assembly surface. Components and connectors can be mounted to the PCBs 8-10 using a fully automated assembly line. Consequently, the configuration is well-suited to mass production.

Once the PCBs 8-10 and the flexible electrical connectors 11, 12 are assembled and the electrical connections to the components are soldered, the battery 16 is attached to the main PCB 8, as shown in Figure 6.

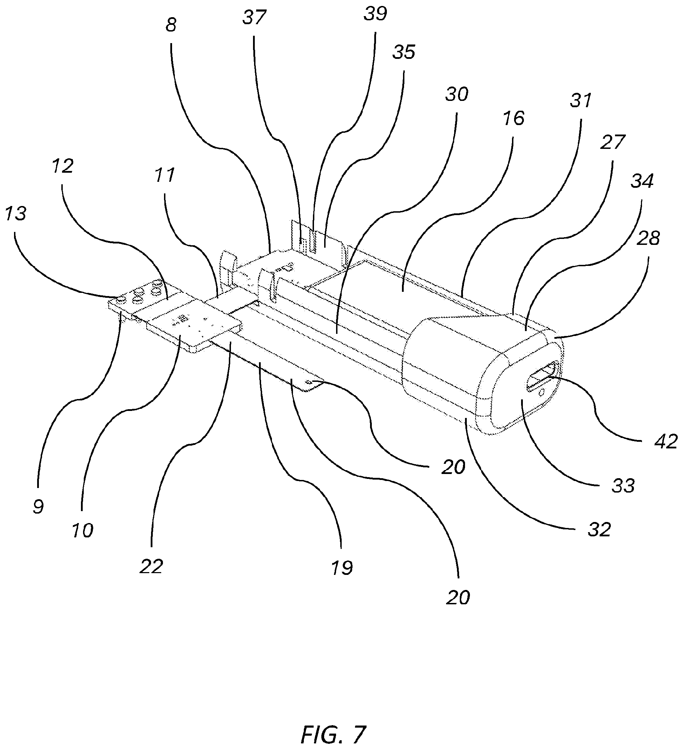

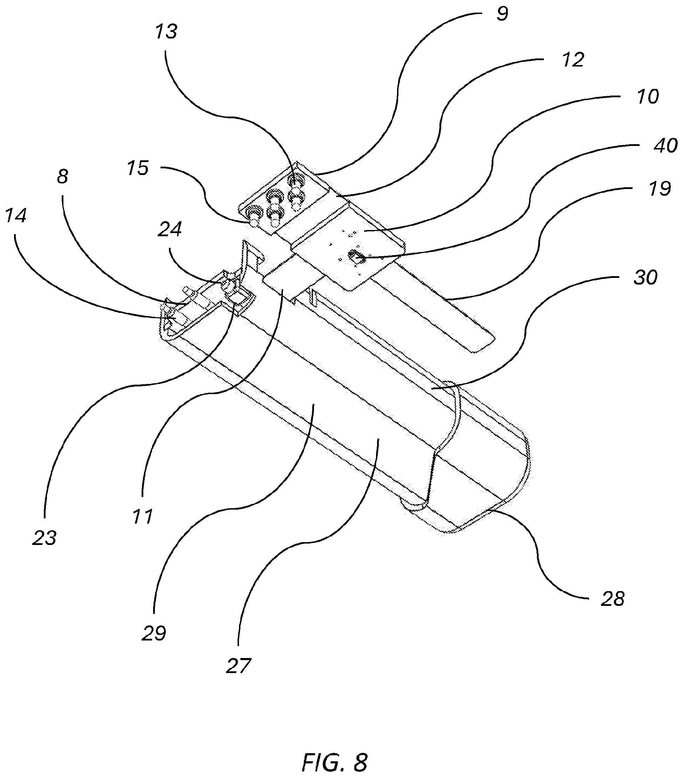

The main PCB 8 and the battery 16 are then inserted into the driver shell 27 with the second flexible electrical connector 11 protruding outwardly from one side of the driver shell 27, as shown in Figures 7 and 8. As can be seen in Figure 8, the drive signal connectors 14 and the airflow sensor port 24 are positioned on the lower, second, side of the main PCB 8.

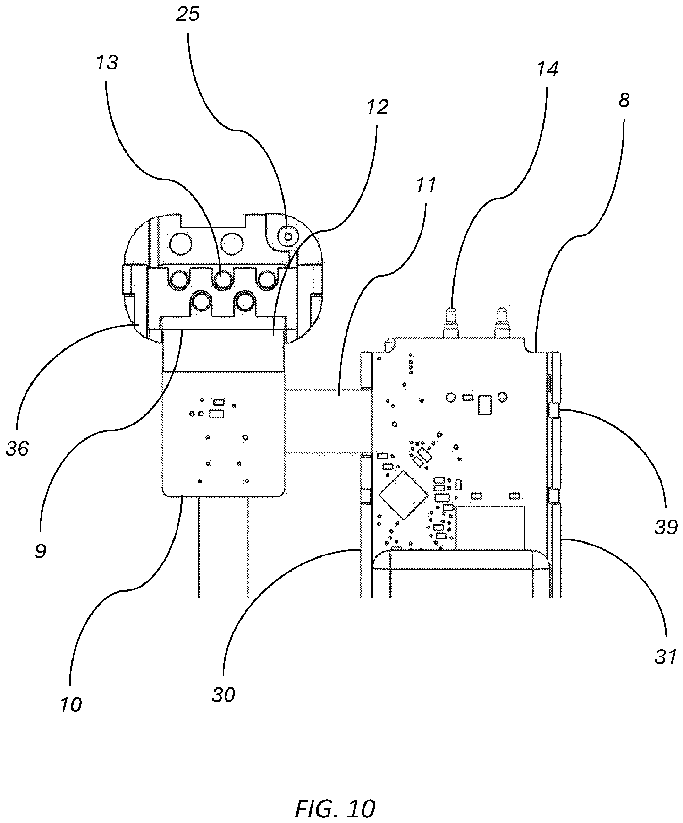

The connector PCB holder 36 is then coupled to the connector PCB 9 by inserting the edges of the connector PCB 9 into corresponding recesses in the connector PCB holder 36, as shown in Figures 9 and 10.

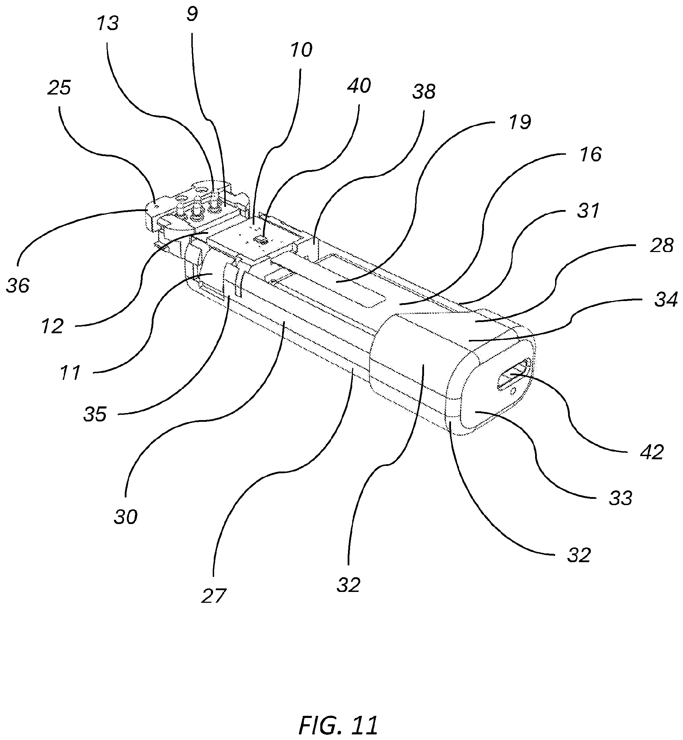

A further PCB holder 38 is then placed over a portion of the main PCB 8 and the further PCB 10 is moved into a position in which the further PCB 10 is superimposed on the further PCB holder 38, as shown in Figure 11. The second flexible electrical connector 11 flexes as the further PCB 10 is moved to enable the further PCB 10 to be located securely in position on the further PCB holder 38. At this point, the connector PCB 9 is positioned in a plane parallel or generally parallel to a plane of the main PCB 8, with the control signal connectors 13 projecting upwardly in a direction transverse, perpendicular to or generally perpendicular to the plane of the main PCB 8, as shown in Figure 11.

Next, the connector PCB holder 36 is pivoted by 90 degrees and attached to the body 35 by the interference fit attachment. The second flexible electrical connector 12 flexes to permit this movement while maintaining the electrical connection between the connector PCB 9 and the further PCB 10. Once in position against the end of the driver shell 27, the drive signal connectors 14 project through respective apertures in the connector PCB holder 36, as shown in Figure 12.

The connector protector 45 is then superimposed on the connector PCB holder 36 so that the control signal connectors 13 and the drive signal connectors 14 project through the apertures 45 in the connector protector 45, as shown in Figure 13. The magnets 46 are then positioned on either side of the connector PCB holder 36, as shown in Figure 14.

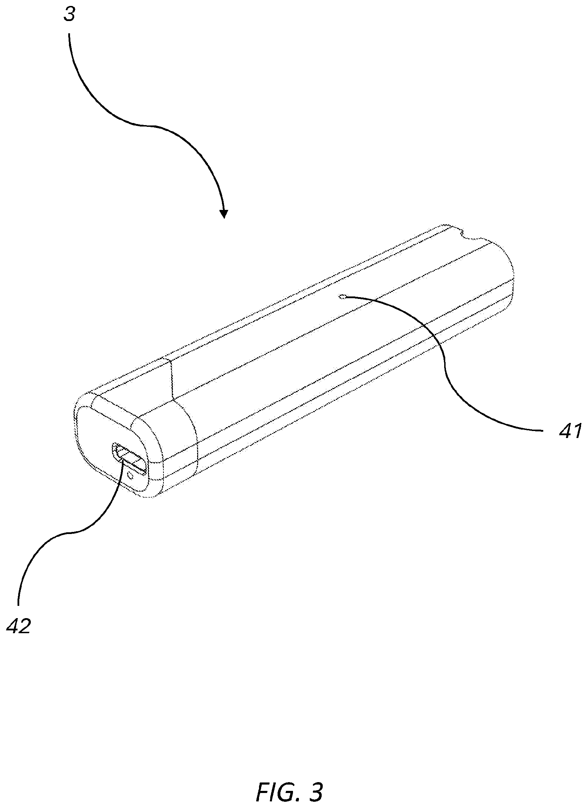

Finally, the driver shell 27 carrying the components of the driver 3 is at least partly inserted into the driver casing 4 so that the driver 3 is fully assembled, as shown in Figure 3.

The configuration of the driver 3 facilitates the method of assembly by enabling the electronic components and connections to be assembled first and then a relatively simple process for moving the PCBs 8-10 and connectors 13, 14 into position. In some examples, the assembly process is fully automated.

The configuration of control signal connectors 13 on the connector PCB 9 with the flexible electrical connector 12 allows for the control signal connectors 13 to be aligned easily with the drive signal connectors 14 so that the contact surfaces 15 of the control signal connectors 13 and the drive signal connectors 14 terminate in the contact plane. Conventional approaches that attempt to align seven pins in a stacked configuration are more difficult to perform and are more likely to suffer alignment problems during manufacture.

The foregoing outlines features of several examples or embodiments so that those of ordinary skill in the art may better understand various aspects of the present disclosure. Those of ordinary skill in the art should appreciate that they may readily use the present disclosure as a basis for designing or modifying other processes and structures for carrying out the same purposes and/or achieving the same advantages of various examples or embodiments introduced herein. Those of ordinary skill in the art should also realise that such equivalent constructions do not depart from the spirit and scope of the present disclosure, and that they may make various changes, substitutions, and alterations herein without departing from the spirit and scope of the present disclosure.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

Although certain example embodiments of the invention have been described, the scope of the appended claims is not intended to be limited solely to these embodiments. The claims are to be construed literally, purposively, and/or to encompass equivalents.

This divisional application is divided from 24827108.2 filed on 6 December 2024 at the European Patent Office (the 'parent application') and the divisional specification as filed comprises the content of the parent application, including, but not limited to, the description, any drawings, any sequence listing(s) and the original claims recited as 'representative features'. The scope of this disclosure therefore includes the full content of the parent application. In any case, protection may be sought for any features disclosed in the parent application as filed.

## Claims

1. A PCB for a driver for use with a mist generator cartridge, the PCB comprising:
a first portion (8) carrying electronic components configured to generate control signals to control the operation of the mist generator cartridge (2) and drive signals to drive the mist generator cartridge (2) to generate a mist, the first portion (8) being provided with a plurality of drive signal connectors (14) each configured to connect electrically to a respective drive signal contact (423) provided on the mist generator cartridge (2) to communicate the drive signals to the mist generator cartridge (2);
a second portion (9) configured to connect electrically to the mist generator cartridge (2);
a third portion (11) positioned between the first portion (8) and the second portion (9), wherein the third portion (11) includes conducting tracks providing an electrical connection between the first portion (8) and the second portion (9) to communicate the control signals between the first portion (8) and the second portion (9),
the first portion (8) being in a first plane and the second portion (9) being in a second plane, the second plane being transverse to the first plane,
wherein the drive signal connectors (14) each extend from the first portion (8) such that a longitudinal axis of each drive signal connector (14) is parallel to the first plane, such that when the mist generator cartridge (2) is coupled to the driver, the drive signals and the control signals are communicated from the driver (3) to the mist generator cartridge (2) to drive and control the operation of the mist generator cartridge (2).

2. The PCB of claim 1, wherein the drive signal connectors (14) are positioned on a first side of the first plane.

3. The PCB of claim 1 or claim 2, wherein the drive signal connectors (14) are spring-loaded pin connectors.

4. The PCB of any one of the preceding claims, wherein each drive signal connector (14) is electrically connected to a conductive track of the first portion (8) along at least part of a side of the drive signal connector (14) with the side of the drive signal connector (14) being parallel to a plane of the first portion (8).

5. The PCB of any one of the preceding claims, wherein the third portion is a flexible PCB.

6. A driver (3) for use with a mist generator cartridge (2), the driver (3) comprising a PCB according to any one of the preceding claims.

7. The driver (3) of claim 6, wherein the driver (3) further comprises:
a driver casing (4) incorporating a casing recess (6) and an open end (7); and
a driver shell (27) carrying the PCB, the driver shell (27) positioned at least partly within the casing recess (6).

8. The driver (3) of claim 7, wherein the drive signal connectors (14) are each elongate with a contact surface at one end, the driver shell (27) carrying the first portion (8) and the second portion (9) of the PCB in a position relative to one another with the contact surfaces of the drive signal connectors (14) terminating in a contact plane to align with the drive signal contacts (423) in a plane on the mist generator cartridge (2).

9. The driver (3) of any one of claims 6 to 8, wherein the driver (3) comprises a connector protector (44) which is resiliently deformable and which is provided with a plurality of apertures (45), wherein the drive signal connectors (14) extend through a respective one of the plurality of apertures (45) with the contact surface of the drive signal connector (14) projecting outwardly from the connector protector (44).

10. The driver (3) of any one of claims 6 to 9, wherein the driver comprises at least one magnet (46) positioned to attract a metal portion of the mist generator cartridge (2) when part of the mist generator cartridge (2) extends into the driver casing (4) through the open end (7).

11. The driver (3) of any one of claims 6 to 10, further comprising:
an airflow sensor (23) which is mounted on the first portion (8); and
an airflow sensor port (24) which is coupled to the airflow sensor (23) and which provides an airflow passage from the airflow sensor (23) to an airflow connector (25), wherein the airflow connector (25) comprises an aperture (26) that is in a plane parallel to the second plane and positioned to connect to an airflow channel (207) on the mist generator cartridge (2) when the mist generator cartridge (2) is coupled to the driver (3).

12. The driver (3) of any one of claims 6 to 11, wherein the driver casing (4) comprises a further open end which is at least partly closed by a portion of the driver shell (27).

13. A mist generator for generating a mist for inhalation by a user, the mist generator comprising:
a driver (3) according to any one of claims 6 to 10; and
a mist generator cartridge (2) coupled to the driver (3).
